# EUROPEAN PATENT APPLICATION

(11) **EP 1 350 519 A1**
(43) Date of publication of application: **08.10.2003**
(21) Application number: 01997190.2
(22) Date of filing: 22.11.2001
(51) Int. Cl.: A61K 38/40, A61K 38/43, A61K 38/46, A61K 38/55, A61K 39/395, A61K 9/14, A61K 9/16, A61K 9/48, A61K 47/42, A61K 47/38, A61K 47/32, A61K 47/46, A61P 29/00, A61P 1/00, A61P 3/04, A61P 9/12, A61P 35/00, A61P 31/12, A61P 31/04, A61P 37/06, A61P 3/10

(54) **DRUGS AND FOODS IMPROVING THE QUALITY OF LIFE AND PROCESS FOR PRODUCING THE SAME**

(30) Priority: 24.11.2000 JP 2000357573
(71) Applicant: Nuclear Receptor Ligand Co., Ltd, Kawasaki-shi, Kanagawa 213-0012 (JP)
(72) Inventor: ANDO, Kunio, Kawasaki-shi, Kanagawa 213-0033 (JP); SUZUKI, Seikichi, Kawasaki-shi, Kanagawa 215-0000 (JP); FUKUDA, Akio, Meguro-ku, Tokyo 153-0061 (JP); KAWAI, Juri, Chiyoda-ku, Tokyo 102-0074 (JP); OGASAWARA, Toshichika, Hamamatsu-shi, Shizuoka 431-2103 (JP); IGUSA, Kazuo, Hamamatsu-shi, Shizuoka 431-2103 (JP)
(74) Representative: Behnisch, Werner, Dr.
(86) International application number: JP0110212
(87) International publication number: WO02041912

(57) **Abstract**

The present invention relates to drugs and foods produced by applying a method whereby a biological activity can be efficiently exerted in the case of orally administering a biologically active protein.

The oral composition according to the present invention contains at least one biologically active protein as the active ingredient and has a means of controlling the release. According to the composition of the present invention, at least 10% of the active ingredient is delivered to the lower digestive tract while sustaining its activity. Namely, the biologically active protein is not degraded in the stomach but delivered to the small intestine, which makes it possible to ensure the exertion of its drug's effect exert a hitherto unknown effect. It is preferable that the biologically benefical active protein contained in the composition according to the present invention is lactoferrin, immunoglobulin, lysozyme, an amylase, a protease, a lipase or a digestive enzyme inhibitor.

## Description

### TECHNICAL FIELD

This invention relates to drugs and foods produced by applying a method whereby a biological activity can be efficiently exerted in the case of a biologically active protein is orally administered. Namely, the present invention relates to drugs and foods wherein orally administered biologically active proteins passe through the stomach without being activated and reach the digestive tract lower than the duodenum (i.e., the action site) while sustaining its biological activity. Further, the present invention relates to diseases which can be newly treated/prevented by these drugs or foods. The method according to the present invention, wherein a biologically active protein can be delivered to the action site without being degraded, is highly efficacious for exerting biological properties.

### BACKGROUND ART

The small intestinal mucosa, which controls the digestion and absorption of foods, has fine villi covering its surface and the villi are further coated with smaller villi. Owing to this structure, the surface area of the mucosa is extremely enlarged. According to one estimate, the surface area of the human digestive tract is 1.5 times larger than a tennis court and the small intestine accounts for major part. It is said that 150 trillion enteric gastrointestinal microflora inhabit the inner cavity of the gastrointestinal tract which can be regarded as outside of the living body. Namely, the human body is in contact with the outside over such a large area and nothing but a thin mucous layer is provided as a partition wall between the body and the outside. This thin mucous layer seemingly responds to various external stimulations. However, it is almost unclear how the mucosa responds to a stimulus coming into contact with it, which part of the mucosa accepts a stimulation, and so on. That is to say, the stimulation-response mechanism of the mucosa still remains unknown. Anyway, it is true that the digestive tract mucosa plays an extremely important role *in vivo.*

As a good example of the response of the intestinal mucosa to a stimulation, the function of the mucosa as a barrier against the invasion of intestinal microflora may be cited. That is to say, intestinal microflora would scarcely invade beyond the barrier so long as an animal is in good health. Once an animal suffers stress due to a surgical operation, trauma, burn, etc. or dies, the intestinal microflora immediately begin to invade into its body beyond the mucous layer. In the normal state, it is therefore supposed that some protection mechanism operates to prevent the intestinal microflora continuously trying to invade. In addition to the intestinal microflora, it is considered that the intestinal mucosa is stimulated by viruses, toxins present in foods and so on. Although it is considered that the intestinal mucosa having such a large area should carry some sensors receiving stimulation and reactors reacting when the sensors receive stimuli, it is scarcely known what kinds of sensors operate therein. For example, it has been clarified that a lactoferrin receptor exists in the intestinal mucosa. However, it has never been known how lactoferrin reacts after binding to the receptor.

Due to the secretion of gastric acid, the inner cavity of the stomach is maintained strongly acidic, i.e., pH 1.0 to 3.0. The strong acidity in the stomach greatly contributes to the sterilization against pathogenic microorganisms contaminating foods or drinking water taken into the stomach. Moreover, the gastric juice contains acidic protease, pepsin so that the stomach is maintained at the optimum pH level for pepsin and proteins are hydrolyzed into peptides fragments. It may be said that most pathogenic microorganisms contaminating foods are exterminated by the pepsin under strongly acidic conditions. That is to say, the stomach serves as the first barrier to fight against the invasion of pathogenic microorganisms. However, in the case where a biologically active protein is orally taken so as to exert its beneficial effect, the degradation in the stomach becomes the largest obstacle. This is because, when the biologically active protein flows into the stomach, its folded three-dimensional structure is broken down under acidic condition and the biologically active protein is hydrolyzed into peptide fragments by the pepsin. As a result, it is assumed that the biological activity of the protein is completely lost.

### DISCLOSURE OF THE INVENTION

To assist the digestion and absorption of foods, it has been a practice to orally administer hydrolases such as lactases, amylases, proteases or lipases. However, it is doubtful whether or not these hydrolases actually hydrolyze foods and assist the digestion and absorption in the human intestine. This is because, when these hydrolases are dissolved at a concentration of 1% in an artificial gastric acid (pH 1.2) containing 50 µg/ml of pepsin and incubated at 37°C for 1.5 hours, their enzymatic activities are completely lost. Since foods are estimated as being retained usually in the stomach for about 1.5 to 2.0 hours, it is obvious that no effect of improving pathological conditions can be expected by the oral administration of these hydrolases.

Also, it has been a practice to orally administer biologically active proteins having an anti-inflammatory effect (for example, serratio-peptidase) or lowering serum cholesterol level (for example, elastase) as a drug. However, it is obvious that these biologically active proteins are also degraded and inactivated when dissolved in the stomach, as in the case of the hydrolases.

Further, egg lysozyme is added as the active ingredient to most widely used combination cold remedies in Japan. Namely, it is a popular basic ingredient of cold remedies in Japan. Lysozyme, which is contained in bodily secretions coating mucosae such as lacrimal fluid, saliva and digestive fluids, has an enzymatic activity of hydrolyzing muramyl peptides constituting bacterial cell wall. Thus, it was once considered as a bactericidal enzyme. When it is orally administered to a patient suffering from a cold, however, it would surprisingly exert an analgesic and anti-inflammatory effect on acute inflammation such as that associated with a sore throat. As a matter of course, it is speculated that a receptor capable of binding to lysozyme exists on the mucous surface and an opioid (a biological narcotic) is released after the binding of lysozyme. However, this mechanism has never been experimentally confirmed. Since lysozyme displays the analgesic and anti-inflammatory effect as described above but has no toxicity on mammals, it has become widely used without side effect as the active ingredient which can be employed in cold remedies. It is considered that lysozyme exerts its analgesic and anti-inflammatory effect in the small intestine. However, further work is required on this point too. In this case, it is also unavoidable that lysozyme is hydrolyzed by pepsin after the disintegration of a tablet in the stomach. Accordingly, it is estimated that only a small portion of lysozyme can avoid being hydrolyzed by pepsin and flow into the small intestine.

In addition, relatively larger proteins such as immunoglobulin and lactoferrin are taken as foods for theirhealth-promoting benefits. Concerning the former, it is recommended from the viewpoint of health promotion to take a skimmed immune milk powder which is prepared by immunizing a milking cow with 26 kinds of intestinal microflora of mice, thus allowing the animal to secret an antibody (IgG1) against the bacterial antigens into the milk and then skimming the milk. However, the immunoglobulin IgG1 contained in the bovine milk is sensitive to pepsin and thus mostly degraded in the course of gastric digestion. It is therefore doubtful that any health-promoting benefits can be thus achieved.

Moreover, it is prohibited to collect bovine colostrum secreted within 1 week after calving. The colostrum is different in quality from ordinary milk secreted more than 1 week after calving. Namely, it contains much immunoglobulin IgG1 and thus solidifies to give a cured after pasteurization or high-temperature short-time processing. Thus, it cannot be pasteurized. In some countries, skimmed colostrum powder which is prepared by skimming the colostrum and spray-drying at a low temperature so as to prevent the immunoglobulin from denaturation is sold commercially as a health-promoting foods. For example, skimmed colostrum powder has been on the market in the United States as a health food under a catch phrase claiming that it relieves arthritis and hypertension. In this case, the immunoglobulin is mostly degraded in the stomach similar to the immune milk as described above.

In recent years, lactoferrin extracting from bovine milk, which is reported to be efficacious in preventing the onset of experimental colon cancer, has attracted considerable public attention as a substance for chemo-preventing cancer. Furthermore, it is reported that the oral administration of a large mount of bovine milk lactoferrin results in the improvement in the pathological conditions of mild hepatitis C patients showing low blood virus titers. Hepatitis C advances into cirrhosis and hepatoma being causative of death in 80% of the carriers. Therefore, the confirmation of the results of this study in other medical fields, if possible, must be good news for patients. On the other hand, lactoferrin should be administered in a dose as large as 3.6 g per day in this study, which obstructs the practical use because of its expensiveness. When the dose is decreased to 1.8 g per day, no significant improvement is observed in the pathological conditions. In this case, there is no doubt that the orally administered lactoferrin is mostly hydrolyzed in the stomach. Accordingly, attempts should be made to decrease the dose by carrying out clinical tests using a preparation which is not hydrolyzed in the stomach and releases no lactoferrin until it flows into the duodenum. At the same time, attempts should be also made to improve the preparation so that even severe hepatitis C can be improved.

Although the total surface area of the digestive tract is 1.5 times as large as a tennis court, whereas the mucosal area of the stomach is very small. Moreover, the stomach plays little role for absorption neither nutrients nor drugs and it has never been reported that any biologically active protein (for example, lysozyme, immunoglobulin or lactoferrin) binds to the gastric surface receptors to thereby improve the pathological conditions. Since the gastric wall is coated with a thick mucous layer, it can be scarcely expected that such a biologically active protein passes through the mucous layer before the hydrolysis by the pepsin and reaches to the mucosa. In the case of orally administering a biologically active protein to exert its drug's effect, the degradation and inactivation in the stomach are the most serious obstacles. Unless these problems are avoided, no biologically active protein should be clinically used in drugs or foods for promoting health.

The present inventors have conducted extensive studies over a long period of time on the oral administration of enzymes, protease inhibitors and biologically active proteins for improving pathological conditions and promoting health. This is because proteins generally have a high safety record and are ideal as remedies so long as the drug effects thereof can be achieved through oral administration, though there are some exceptions such as lysine. Discussions have been made mainly on lactoferrin and immunoglobulin. Lactoferrin, which is an ferric iron-binding protein having a molecular weight of about 80,000, seemingly causes changes *in vivo* when brought into contact with its receptor mucosa. One of these changes is to enhance the immune potency against infection to pathogenic microorganisms. Considering these effects, it is assumed that there is a structure such as a so-called lactoferrin receptor on the mucosa, though the detailed mechanism thereof still remains almost unknown.

When such a protein is orally administered to animals to study its biological activity, the effect is exhibited in some cases but not in others even though the administration dose and conditions are identical. That is, only an extremely poor reproducibility can be established. For example, 450 mg/kg of 5-fluorouracil (5-FU) is orally administered to BALB/c mice and thus bacteremia is induced due to bacterial translocation (BT), thereby causing the death of the mice from multiple organ failure. In this experimental system, the BT can be inhibited and thus the mice can survive in some cases by orally administering bovine lactoferrin one day before the administration of 5-FU or simultaneously therewith. Moreover, this BT inhibitory effect is statically significant only when administering lactoferrin in a small single dose of 2 mg/kg. If BT caused by the administration of an anticancer agent could be retarded by orally administering 2 mg/kg of lactoferrin, it might be possible to regulate human BT with small dose of lactoferrin induced by surgical operation, cancer, stress and the like and prevent multiple organ failure due to infection and systemic inflammatory response syndrome, which would create a large impact on the medical-care industry.

On the other hand, the above-described effect is not always exerted in other cases even though 100 mg/kg of lactoferrin is orally administered under the similar conditions to shown above. This statement is adequate with the proviso that the present inventors merely consider that the conditions are the same. That is to say, the same phenomenon is not observed since the conditions are different in practice. Investigations on the cause of the poor reproducibility of the BT inhibitory effect clarify that it might be caused by the degradation of lactoferrin in the digestive tract. Namely, a biologically active protein cannot exert its biological effect unless it reaches the action site while completely retaining its structure. Accordingly, it is suggested that lactoferrin is degraded under certain condition before reaching the action site and thus fails to exert its effect.

When a biologically active protein such as lactoferrin is orally administered, therefore, most molecules thereof are significantly degraded during the passage through the stomach and thus loses its effectiveness. Such an orally administered biologically active protein can only exert its effect by chance in the case where a small portion thereof has not been hydrolyzed during the passage through the stomach and flows into the duodenum. Accordingly, the present inventors worked to develop a method wherein a biologically active protein orally administered to improve pathological conditions and promote health can pass through the stomach without degradation and remains intact until it reaches the duodenum.

### DETAILED DESCRIPTION OF THE INVENTION

In the existing preparations, orally administered lactoferrin only comes into transient contact with the mucosae in the oral cavity and the esophagus. After flowing into the stomach, it is seemingly degraded into polypeptide fragments by pepsin, thus losing its effect. This high sensitivity to acid protease has been interfering with the broad application of lactoferrin. Lactoferrin is a trace component of bovine milk and thus relatively expensive. Thus, the most serious weak point thereof resides in that it is highly sensitive to acid protease such as pepsin and papain and easily hydrolyzed thereby. However, it has been found out that lactoferrin is highly resistant against other proteases and is hardly degraded even after being allowed to stand unaerobically at 37°C in feces for over 1 week (see the following Example 4). From this viewpoint, the present inventors assumed that, if orally administered lactoferrin can pass through the stomach without degradation and flow into the duodenum, then it can improve pathological conditions and promote health even in small doses. It is on the above assumption that the present invention has been thus completed.

Accordingly, the present invention provides an oral composition containing as the active ingredient at least one biologically active protein which has a means of controlling the release so that more than at least 10% of the active ingredient is released into the lower digestive tract while sustaining its activity.

The term "oral composition" as used herein involves medicinal compositions and/or foods in any forms which are to be orally taken. The medicinal composition according to the present invention can be in various medicinal preparation forms such as tablets, capsules, pills, fine grains and dusts. The foods include functional foods, health foods and nutrient-supplemented foods.

The composition according to the present invention should have a means of controlling the release. The means of controlling the release as used herein is a means whereby at least 10% (preferably 20% or more, still preferably 50% or more and still preferably 80% or more) of the active ingredient in the composition is prevented from irreversible fragmentation in the stomach (for example, the active ingredient is not released or dissolved and, therefore, not denatured and/or degraded in the stomach) and delivered to the lower digestive tract (the duodenum and small intestine) while keeping its intact structure.

As the means of controlling the release for the composition according to the present invention, use can be made of various means conventionally employed in the art to control the release of drugs. These means include, for example, the method of coating a core containing the active ingredient with an enteric film or a film made of a hydrophobic material and having an appropriate thickness, the method of carrying the active ingredient on a matrix and releasing the drug component under definite conditions, and so on.

The present invention can be applied to any proteinous active ingredients which would be denatured and/or degraded and thus inactivated in the stomach. These active ingredients include biologically active proteins which are inactivated by acid proteases, i.e., pepsin and papain. More specifically speaking, these active ingredients include basic proteins such as lactoferrin and lysozyme showing immunopotentiating, analgesic and anti-inflammatory effects; immunoglobulin inhibiting the growth of pathogenic microorganisms; hydrolases assisting the digestion and absorption of foods in the small intestine; and protease or peptidase inhibitors inhibiting the activity of these hydrolases. Examples of the hydrolases assisting the digestion and absorption of foods include various proteases, amylases and lipases. Lactoferrin, which can be cited as a particularly preferable active ingredient, can exert immediate therapeutic actions on various diseases which have been refractory hitherto (see the therapeutic effects in the following Examples).

The present invention is further usable.for treating systemic inflammatory response syndrome; inflammatory bowel disease; lifestyle-related diseases such as obesity, diabetes or hypertension; malignant tumor; infections with pathogenic viruses including hepatitis C virus, a rotavirus, a herpesvirus or a cytomegarovirus; infections with pathogenic microorganisms; and autoimmune diseases; and/or treating or improving various symptoms accompanying these diseases or conditions that have a negative impact on the quality of life of a patient.

Systemic inflammatory response syndrome, which is a whole body inflammation occurring in patients with various diseases, includes infection, pancreatitis, ischemia, multiple trauma, hemorrhagic shock, immune-mediated organ injury and sepsis, as well as complications (acute lung injury; shock; renal failure; and multiple organ failure, and abnormalities in organ functions) frequently accompanying systemic inflammatory response syndrome. Inflammatory bowel disease includes Crohn's disease and ulcerative colitis.

### MODE FOR CARRYING OUT THE INVENTION

A mode for carrying out the present invention is a composition wherein lactoferrin granules or tablets are surface-coated with an enteric film which is dissolved not in the stomach but after flowing into the intestine. Now, this composition will be described in greater detail.

Either spray-dried or a freeze-dried powder of bovine milk lactoferrin has an extremely low bulk specific gravity and thus is difficult to tablet directly. Furthermore, it is unstable to moisture and high temperature. Owing to these diffficulties, it is preferable to process the powder of bovine milk lactoferrin into a preparation under a dry state. Namely, lactoferrin is mixed with diluents, binders and disintegrants. The obtained mixture is then molded under a high pressure with the use of a slug machine to provide a thin and large flat disk. After, the disk is ground and sieved to give granules of a definite size. To give a product, these granules are then coated with an enteric film and filled into hard capsules in definite portions. In the case of a tablet product, the granules are blended with a lubricant(s) and tabletted followed by coating with an enteric film.

The enteric-coated tablets and granules of lactoferrin thus produced are subjected to a disintegration test by using a first solution (pH 1.2) prepared by dissolving 2.0 g of sodium chloride and 24 ml of dilute hydrochloric acid in water to give a total volume of 1000 ml and a second solution (pH 6.8) prepared by adding 118 ml of a 0.2N sodium hydroxide reagent solution and water to 250 ml of a 0.2M potassium dihydrogen phosphate reagent solution to give a total volume of 1000 ml (see (6) Enteric Preparation, Disintegration Test Method, General Test Method 41 of Pharmacopoea of Japan (9th revision) or General Test Method 47 of Pharmacopoea of Japan (13th revision)). The tablets or granules produced by the method according to the present invention are not disintegrated by immersing the first solution for 120 minutes but disintegrated by immersing in the second solution within 60 minutes. That is to say, it is thus possible to produce a preparation which is neither disintegrated nor dissolved in the stomach but disintegrated after flowing into the duodenum, thereby releasing lactoferrin.

Examples of the diluents to be used in producing the enteric preparation according to the present invention include monosaccharides, disaccharides and polysacoharldes such as lactose, sucrose and glucose, starches such as corn starch and potato starch, crystalline cellulose, inorganic materials such as light silica gel, synthetic aluminum silicate, magnesium metasilicate aluminate and calcium hydrogen phosphate and so on. Examples of the binder include starches, carboxymethylcellulose (CMC), hydroxypropylcellulose (HPC), carboxymethylcellulose sodium salt, polyvinylpyrrolidone and so on. Examples of the disintegrator which are employed to stimulate disintegration the preparation into the original primary particles include starch, carboxymethylcellulose sodium salt, carboxymethylcellulose calcium salt, croscarmellose sodium, carboxymethylstarch sodium and so on. Examples of the film agent for coating the tablets or granules to give an enteric preparation include hydroxypropylmethylcellulose phthalate, carboxymethylethylcellulose, cellulose acetate phthalate and methacrylic acid copolymers which are soluble at pH 5 to 6 and shellac which is soluble in the alkaline region.

The composition according to the present invention generally contains the active ingredient in an amount of from about 0.1 mg to about 100,000 mg per day, preferably from about 1 mg to about 50,000 mg and still preferably from about 10 mg to about 10,000 mg per day. It may be administered either at once or in portions to a patient with a need for the treatment or the improvement in certain conditions. The dose can be individually determined depending on the age and body weight of the patient and the purpose of the administration.

### Examples

### <Example 1 (Experiment using rat ligated at the pylorus: No. 1)>

Using 6 male Wistar rats weighing about 300 g which had fasted overnight, a small incision was made on the abdomen above the pylorus of each animal under ether anesthesia. Then the junction between the pylorus and the duodenum was lightly ligated with a surgical thread so as to prevent the contents of the stomach from flowing toward the duodenum. Next, the incision was quickly sutured. An hour after awakening the rats, 50 mg portions of lactoferrin extracted from bovine milk and immunoglobulin (each manufactured by Tatua Milk Biologics, purity: about 90% and 75% respectively) were dissolved in 5 ml of distilled water and orally administered. 15 minutes, 30 minutes and 1 hour after the administration, 2 rats were sacrificed each time. The contents of the stomach were then washed out into a 300 ml beaker and the stomach wall was sufficiently washed with biological saline. The contents of the stomach were combined with the washing saline and the volume was adjusted accurately to 200 ml, thereby giving a sample solution. The lactoferrin and immunoglobulin IgG1 dissolved in the sample solution were quantified by high performance liquid chromatography.

As the following table shows, 4.6%, a trace and no lactoferrin remained after 15 minutes, 30 minutes and 60 minutes respectively.

**Table 1:**

| Hydrolysis of biologically active proteins in stomach | | |
|---|---|---|
| Time (min) | Lactoferrin (mg/stomach) | IgG1 (stomach) |
| 15 | 2.1 (4.6%) | 3.1(8.3%) |
| 30 | Trace | Trace |
| 60 | 0 | 0 |

Each value in parentheses shows the remaining ratio to the administration dose.

### <Example 2 (Experiment using rat ligated at the pylorus: No. 2)>

Using 6 male Wistar rats weighing about 250 g which had fasted overnight, a small incision was made on the abdomen above the pylorus of each animal under ether anesthesia. Then the junction between the pylorus and the duodenum was lightly ligated with a surgical thread. Next, the incision was quickly sutured. An hour after awakening the rats, 100 mg portions of bovine milk and colostral immunoglobulin (each manufactured by Tatua Milk Biologics) and 5 mg of pepstatin (a pepsin inhibitor) were dissolved in 5 ml of biological saline and orally administered. 15 minutes, 30 minutes and 1 hour after the administration of lactoferrin, 2 rats were sacrificed each time. As in Example 1, the contents of the stomach were washed out and the volume was adjusted accurately to 200 ml. Then the lactoferrin and immunoglobulin IgG1 dissolved therein were quantified by high performance liquid chromatography.

As the following table shows, 53%, 23% and 10% of lactoferrin remained after 15 minutes, 30 minutes and 60 minutes respectively. Compared with Example 1, lactoferrin remained in obviously larger amounts. These results clearly indicate that pepsin participates in the degradation of lactoferrin in the stomach.

**Table 2:**

| Hydrolysis of biologically active proteins in stomach in the presence of pepsin inhibitor | | |
|---|---|---|
| Time (min) | Lactoferrin (mg/stomach) | IgG1 (stomach) |
| 15 | 47.7 (53%) | 15.7 (20.9%) |
| 30 | 20.7 (23%) | 8.3 (11.1%) |
| 60 | 0.9 (10%) | 11.0 (14.7%) |

Each value in parentheses shows the remaining ratio to the administration dose.

### <Example 3>

Using 6 male Wistar rats weighing about 300 g fed on commercial pellet diet and water *ad libitum*, a small incision was made on the abdomen of each animal under ether anesthesia. Then the ileum was ligated at the pylorus and the upper part of the cecum to form a small intestinal loop. Next, 100 mg portions of bovine milk lactoferrin and colostral immunoglobulin (manufactured by Tatua Milk Biologics, purity: about 90%) were dissolved in 5 ml of biological saline and injected into the small intestinal loop with the use of a syringe. After quickly suturing the abdominal incision, the rats were returned to their cage and the loops were taken out 30, 60 and 120 minutes thereafter. Then the contents were transferred into a beaker and the intestinal wall was sufficiently washed with biological saline. The contents were combined with the washing liquor to give a sample solution. The lactoferrin contained in the sample solution was quantified by high performance liquid chromatography. The following table shows the results.

**Table 3:**

| Degradation of biologically active proteins in small intestine | | |
|---|---|---|
| Time (min) | Lactoferrin (mg/stomach) | IgG1 (stomach) |
| 15 | 83 (92%) | 47 (63%) |
| 30 | 89 (99%) | 22 (29%) |
| 60 | 80 (89%) | 7 (9%) |

Each value in parentheses shows the remaining ratio to the administration dose.

As Table 3 shows, lactoferrin was not degraded in the small intestine. However, immunoglobulin IgG1 was slowly degraded showing a half-life of about 30 minutes.

### <Example 4>

10 g of fresh feces of male Wistar rats were added to 50 ml of distilled water and homogenized with the use of a Polytron Homogenizer. To 10 ml of the homogenate were added 100 mg portions of bovine milk lactoferrin and colostral immunoglobulin IgG1 and dissolved therein. After incubating under an anaerobic jar at 37°C for 24, 72 and 168 hours, the lactoferrin and immunoglobulin dissolved therein were measured by high performance liquid chromatography.

As Table 4 shows, lactoferrin was scarcely degraded after incubating together with the feces for 1 week. In contrast, immunoglobulin completely disappeared after incubating for 72 hours. Thus, it can be understood that immunoglobulin IgG1 was largely hydrolyzed by the intestinal microflora while lactoferrin was highly resistant against proteases produced by the intestinal microflora.

**Table 4:**

| Degradation of biologically active proteins by intestinal bacteria | | |
|---|---|---|
| Time (min) | Lactoferrin (mg/stomach) | IgG1 (stomach) |
| 15 | 72 (80%) | 13 (17%) |
| 30 | 70 (78%) | 0 (0%) |
| 60 | 76 (84%) | 0 (0%) |

Each value in parentheses shows the remaining ratio to the administration dose.

### <Example 5>

Enteric capsules (No.1 Pharmacopoea of Japan) were produced by molding a hydroxypropylalkylcellulose (HP-55™ manufactured by Shin-Etsu Chemical Co., Ltd.) which was not dissolved under acidic conditions of pH 3.0 or lower but dissolved under neutral to weakly alkaline conditions. Fine grains of 10 parts of lactoferrin and 5 parts of carboxymethylcellulose calcium were mixed in a dry state without adding water to both and the obtained mixture was molded under a high pressure with the use of a slug machine to give a thin and large flat disk. Then the disk was ground and passed through a 16-mesh sieve to give granules of about 0.1 mm in diameter. 150 mg portions of these granules were filled in the hard capsules and the joint part was sealed with an enteric base. Thus, an enteric lactoferrin hard capsule preparation was obtained.

### <Example 6>

Fine grains were obtained as in Example 5 by using a mixture of 10 parts of skimmed bovine colostrum powder containing 8% of immunoglobulin with 10 parts of microcrystalline cellulose. Then the obtained grains were filled in 150 mg portions into the hard capsules of Example 5 and the joint part was sealed with an enteric coating film. Thus, an enteric-coated skimmed colostrum preparation was obtained.

### <Example 7>

Tablets of 8 mm in diameter each containing 50 mg of lactoferrin were produced by mixing a lactoferrin powder with lactose, cellulose and carboxymethylcellulose calcium salt in a dry state without adding water, high-pressure molding the mixture in the dry state as such with the use of a slug machine to give a thin and large flat disk, grinding the disk and passing through a 16-mesh sieve to give granules of a definite size and then tabletting the granules together with a sucrose fatty acid ester. Using a film of 0.7 mm in thickness prepared by adding water to 10 parts of gelatin and 20 parts of ribonucleic acid sodium and dissolving under heating, an outer coating was formed by the flat plate method. After drying, an enteric-coated preparation was obtained.

### <Example 8>

The tablets (lactoferrin content: 50 mg, diameter: 8 mm, weight: 180 mg) of Example 7 before the formation of the outer coating were put into a coating machine (Hi-Coater HCT-48N manufactured by Freund Industrial Co., Ltd.) and an enteric coating solution comprising 9% of carboxymethylethylcellulose, 1% of a glycerol fatty acid ester, 45% of ethanol and 45% of methylene chloride was sprayed thereto. Thus, the tablets were coated with 12% by weight (based on the tablets) of the enteric coated film to give a product.

### <Example 9>

The tablets (lactoferrin content: 50 mg, diameter: 8 mm, weight: 180 mg) of Example 7 before the formation of the outer coating were put into a coating machine (HC-MINI prepared by dissolving protein obtained from corn kernels, 8 parts of Tween and 2 parts of glycerol in 150 parts of 70% ethanol was sprayed thereto in a calculated amount. Thus, tablets coated with 10% by weight (based on the tablets) of the enteric-coated film were obtained.

### <Example 10>

The tablets (lactoferrin content: 50 mg, diameter: 8 mm, weight: 180 mg) of Example 7 before the formation of the outer coating were put into a coating machine (HC-MINI manufactured by Freund Industrial Co., Ltd.) and a solution prepared by dissolving 30 parts of shellac and 7 parts of castor oil in 63 parts of isopropanol was sprayed thereto in a calculated amount. Thus, tablets coated with 10% by weight (based on the tablets) with the enteric-coated film were obtained.

### <Therapeutic effects>

The thus produced enteric compositions have remarkable effects. For example, the enteric lactoferrin preparations exert immediate therapeutic actions on various diseases which have been hardly treated hitherto.
(1) When patients are infected with hepatitis C via blood transfusion, they suffer from acute hepatitis which then progresses into chronic hepatitis and cirrhosis. Finally, there arises hepatic cancer which causes death. It is estimated that hepatitis C progresses into hepatic cancer at a high ratio of about 80%. Oral administration of an enteric lactoferrin preparation (produced in Example 8 or 9 as described above) to a hospitalized patient suffering from hepatic cancer and abdominal fluid retention resulted in astonishing therapeutic effects. By orally administering only 0.9 g/day of lactoferrin, the abdominal fluid was absorbed and eliminated and amelioration in icterus and a large decrease in the hepatitis C virus marker in blood were observed after 3 days. This patient, who had been believed by the doctor in charge to have only several months to live, was released from hospital and was able to walk without assistance.
(2) In the case of a patient suffering from malignant lymphoma, cancer was controlled by chemotherapy. However, because of the frequent occurrence of side effects of the chemotherapy, he had been in sickbed continually. Although he took various drugs in an attempt to remedy the situation, no satisfactory effect could be obtained. He even tried various folk medicines, but no improvement in the symptoms was achieved thereby. However, after orally taking enteric lactoferrin tablets produced in Example 8 or 9 as described above (lactoferrin dose: 0.45 g per day) for 2 days, he began to develop an appetite, and after 3 days, his clinical test data showed remarkable improvements, thus surprising the doctor in charge. In the case of this patient, the quality of life, which had been seriously worsened due to lassitude, vomiting, frequent onset of infection, inappetence, etc., was significantly improved by the oral administration of the enteric lactoferrin tablets.
(3) Enteric lactoferrin tablets were administered to a patient suffering from a recurrence of gastric cancer after a surgical operation and retention of cancerous abdominal fluid. In this case, the abdominal fluid was drawn several times a week to relieve pain. After orally taking enteric lactoferrin tablets produced in Example 8 or 9 as described above (lactoferrin dose: 0.45 g per day) for 1 week, however, the abdominal fluid was completely absorbed and eliminated. Although this patient had displayed typical symptoms of systemic inflammatory response syndrome (SIRS), i.e., continuous slight fever, a heart rate of 95 per minute and a respiration rate of 23 per minute, these symptoms disappeared after taking the enteric lactoferrin tablets for 1 week; the patient's appetite also returned.
(4) To a female patient who had suffered from atopic dermatitis for 13 years and could not sleep well at night due to systemic itching, enteric lactoferrin tablets produced in Example 8 or 9 as described above (lactoferrin dose: 0.45 g per day) were orally administered. From day 2 after the initiation of the administration, the rash of atopic dermatitis was relieved, the itching stopped and she was able to sleep well. That is to say, lactoferrin exhibited an effect of freeing a patient from stress due toautoimmune disease. When enteric lactoferrin tablets were administered to a patient having a decrease in saliva secretion due to Soegren's syndrome, the pH value of the saliva was elevated and the secretion was promoted. Thus, the dryness in the oral cavity was considerably relieved. It could be concluded, therefore, that lactoferrin is obviously efficacious in improving a quality of life worsened by autoimmune diseases.
(5) As discussed above, some biologically active proteins exert the effects thereof in the digestive tract lower than the duodenum in many cases. Although the discussion has focused mainly on lactoferrin herein, there are biologically active proteins other than lactoferrin which can exhibit effects unknown hitherto or the administration dose of which can be largely reduced, when they pass through the stomach without any changes and remain un-dissolved until flowing into the small intestine. Immunoglobulin and lysozyme are proteins showing similar effects to lactoferrin when processed into enteric preparations. Also, amylases, proteases and lipases to be orally administered for assisting the digestion and absorption of foods should be processed into enteric preparations, since they exert effects in the small intestine. Furthermore, it is noteworthy that obesity frequently observed in people of modern times can be prevented by administering after ingesting inhibitors for digestive enzyme secreted *in vivo*, since the absorption of nutrients can be thus regulated. It has been clarified that certain proteins originating in plants inhibit digestive enzymes secreted *in vivo.* Therefore, obesity can be prevented and thus lifestyle-related diseases such as type II diabetes, hypertension and arteriosclerosis can obviously be improved by orally administering after ingesting inhibitors for amylases, lipases and proteases in the form of enteric preparations. Namely, the present invention is applicable not only to lactoferrin but also biologically active proteins over a broad range.

## Claims

1. An oral composition containing as the active ingredient at least one biologically active protein selected from the group consisting of enzymes, lactoferrin, immunoglobulin, lysozyme, amylase inhibitors, lipase inhibitors and protease inhibitors, which has a means of controlling the release so that at least 10% of the active ingredient is released into the lower digestive tract while sustaining its activity.

2. An oral composition according to claim 1 wherein the active ingredient is lactoferrin.

3. An oral composition according to claim 1 or 2 which is to be used for treating or improving systemic inflammatory response syndrome; inflammatory bowel disease; lifestyle-related diseases such as obesity, diabetes or hypertension; malignant tumor; infections with pathogenic viruses including hepatitis C virus, a rotavirus, a herpesvirus or a cytomegarovirus; infections with pathogenic microorganisms; and autoimmune diseases; and/or symptoms accompanying these diseases or conditions.

4. An oral composition containing as the active ingredient at least one biologically active protein selected from the group consisting of enzymes, lactoferrin, immunoglobulin, lysozyme, amylase inhibitors, lipase inhibitors and protease inhibitors, which has a means of controlling the release and in which the means of controlling the release contains an enteric-coated film.

5. An oral composition according to claim 4 wherein the active ingredient is lactoferrin.

6. An oral composition according to claim 4 or 5 which is to be used for treating or improving systemic inflammatory response syndrome; inflammatory bowel disease; lifestyle-related diseases such as obesity, diabetes or hypertension; malignant tumor; infections with pathogenic viruses including hepatitis C virus, a rotavirus, a herpesvirus or a cytomegarovirus; infections with pathogenic microorganisms; and autoimmune diseases; and/or symptoms accompanying these diseases or conditions.

7. An oral composition according to any of claims 4 to 6 wherein the enteric-coated film contains a materials selected from the group consisting of shellac, Tween, phospholipids, hydroxypropylmethylcellulose phthalate, carboxymethylethylcellulose, cellulose acetate phthalate, methacrylic copolymers, water-insoluble ethylcellulose and aminoalkyl methacrylate copolymers.

8. An oral composition according to claim 4 containing lactoferrin as the active ingredient and being in the form of dusts, granules, capsules containing a definite amount of dusts or granules enclosed therein or tablets, which can be obtained by a production method comprising the following steps:
mixing a lactoferrin powder with additives acceptable either pharmaceutically or dietary in a dry state;
if desired, high-pressure molding the mixture in a dry state and then processing the molded article into fine grains or granules of a definite size; and
coating the fine grains or granules with an enteric film or tabletting the mixture, fine grains or granules and then coating the tablets with the enteric film.

9. A method of producing an enteric oral composition in the form of dusts, granules, capsules containing a definite amount of dusts or granules enclosed therein or tablets which comprises the following steps:
mixing a lactoferrin powder with additives acceptable either pharmaceutically or dietary in a dry state;
if desired, high-pressure molding the mixture in a dry state and then processing the molded article into fine grains or granules of a definite size; and
coating the fine grains or granules with an enteric-coated film or tabletting the mixture, fine grains or granules and then coating the tablets with the enteric-coated film.

10. An oral composition according to claim 4 which contains lactoferrin as the active ingredient and complies with the requirement as defined in the disintegration test for enteric preparations specified in General Tests of Pharmacopoea of Japan.
